# EUROPEAN PATENT SPECIFICATION

(11) **EP 2 775 920 B1**
(45) Date of publication and mention of the grant of the patent: **17.08.2016**
(21) Application number: 12788005.2
(22) Date of filing: 01.11.2012
(51) Int. Cl.: A61B 5/15, A61B 5/151

(54) **LANCING DEVICE WITH CAM-ACTUATED DRIVE AND SEPARATE GUIDANCE**
LANZETTENVORRICHTUNG MIT NOCKENBETÄTIGTEM ANTRIEB UND GETRENNTER FÜHRUNG
AUTOPIQUEUR AYANT UN ÉLÉMENT D'ENTRAÎNEMENT ACTIONNÉ PAR CAME ET UN ÉLÉMENT DE GUIDAGE SÉPARÉ

(30) Priority: 08.11.2011 US 201161556897 P
(43) Date of publication of application: 17.09.2014
(73) Proprietor: Facet Technologies, LLC, Kennesaw, GA 30144 (US)
(72) Inventor: VINE, Douglas, A., Alpharetta, GA 30004 (US)
(74) Representative: Maschio, Antonio
(86) International application number: PCT/US2012/063015
(87) International publication number: WO 2013/070488

(56) References cited:
- EP-A1- 2 218 392
- EP-A2- 1 090 584
- EP-A2- 1 779 781
- WO-A1-2006/110573
- WO-A1-2008/107382
- WO-A2-2006/058654

## Description

### Technical Field

The present invention relates generally to the field of medical devices, and more particularly to an improved lancing device for blood testing and typing applications.

### Background

In typical known lancets, a needle (lance) is affixed to some sort of movable needle carrier, such as a sled or carriage. A drive spring urges the needle carrier outwardly toward an extended position in which the needle extends beyond the housing to penetrate the skin or tissue of the user. Another spring is positioned for urging the needle carrier inwardly toward a retracted position in which the needle withdrawn and does not extend beyond the housing. In such a device it is common to try to balance these springs more or less to try to avoid "bouncing" of the needle and a double strike of the user by the lancing device. This balancing effort can be difficult. Typically, these springs, which tend to urge the needles toward extended and retracted positions, are acting on the same part of the lancing device (normally the carrier). Having the drive springs act on parts that are also involved in guidance tends to distort the guidance efforts, as well as the drive efforts. Thus, by having one mechanism involved in both functions, both functions are diminished somewhat.

EP 1 090 584 discloses a lancet unit for taking blood samples for diagnostic purposes comprising a spring and a rotor which has a working surface with vertices at a maximum distance from its axis of rotation, and connected to a lancet holder in such a way that the lancet point emerges from the lancet opening while the rotor relative to the spring roller is within the angular thrust zone following each vertex.

WO 2006/058654 discloses a pricking device for taking blood, comprising an element for contacting an extraction surface, an actuating element for manually actuating the pricking device, and a pricking element that is movable relative to the contacting element substantially perpendicular to the extraction surface and is used for perforating the extraction surface.

WO 2008/107382 discloses a pricking device for taking blood for medical tests, comprising a base, at least one needle arranged therein, of which a pointed end can be deployed, and which is provided with a needle retaining element that at least partly embraces the needle, and a manual actuating element for inducing a movement of the needle along the needle retaining element relative to the base. The needle is spring-loaded.

WO2006/110573 discloses a lancet device including a housing and a lancet structure having a puncturing tip maintained with an interior cavity of the housing. The device incorporates a bow spring which cooperates with the lancet structure within the housing, and a trigger which extends laterally through and into the housing. The bow spring is resistive to movement, and therefore maintains the puncturing tip in a retracted position within the housing. Lateral movement of the trigger into the housing, such as through a pivoting trigger operation, biases the bow spring against this resistive force to deflect the bow spring an extend the puncturing tip through the forward end of the housing to achieve a puncturing position.

EP 1 779 781 discloses an analyte monitoring system comprising an external system housing, a lancing apparatus and a meter for determination of the analyte. The lancing apparatus includes an inner housing, a firing mechanism, a lancing mechanism and a linkage arm. The firing mechanism and lancing mechanism are configured to produce a firing force and a lancing force respectively in opposing directions, and the linkage arm is configured to convert the firing force into the lancing force.

### Summary

In accordance with the present invention, there is provided a lancet assembly as defined in claim 1.

Further advantages are achieved by the embodiments indicated by the dependent claims.

These and other aspects, features and advantages of the invention will be understood with reference to the drawing figures and detailed description herein, and will be realized by means of the various elements and combinations particularly pointed out in the appended claims. It is to be understood that both the foregoing general description and the following brief description of the drawings and detailed description of the invention are exemplary and explanatory of preferred embodiments of the invention, and are not restrictive of the invention, as claimed.

### Brief Description of the Drawings

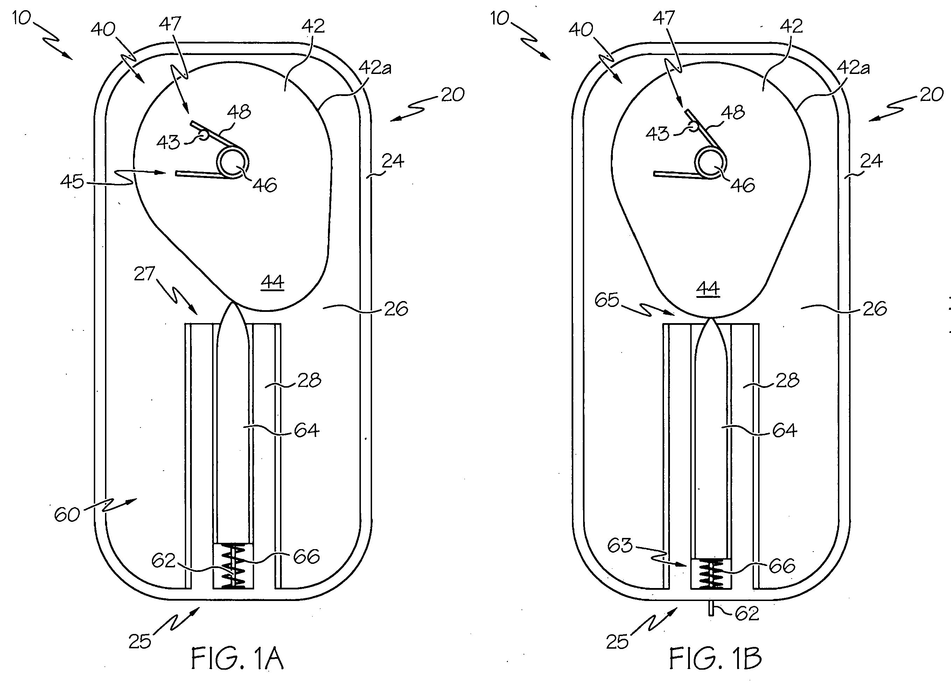

**FIGURE 1A** is a side view of a lancing device with cam-actuated drive and separate guidance according to a first example embodiment of the present invention.

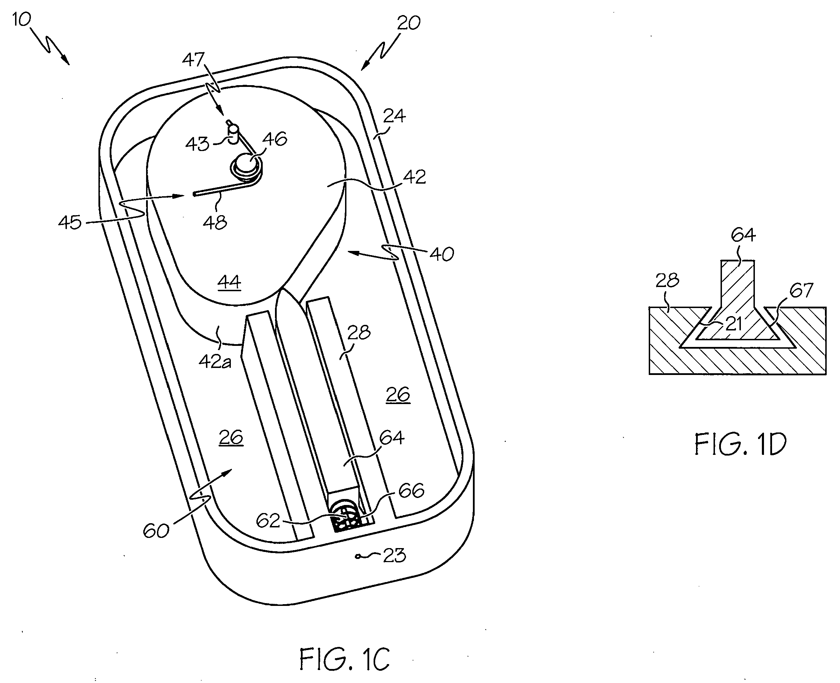

**FIGURE 1** **B** is another side view of the lancing device with cam-actuated drive and separate guidance of **Figure 1A****.**
**FIGURE 1C** is a perspective view of the lancing device with cam-actuated drive and separate guidance of **Figure 1A****.**
**FIGURE 1** **D** is a side view of the lancing device guide surface and needle carriage according to a preferred embodiment of the present invention.
**FIGURE 2A** is a side view of a lancing device with cam-actuated drive and separate guidance according to another example embodiment.
**FIGURE 2B** is another side view of the lancing device with cam-actuated drive and separate guidance of **Figure 2A****.**
**FIGURE 3A** is a side view of a lancing device with cam-actuated drive and separate guidance according to another example embodiment.
**FIGURE 3B** is another side view of the lancing device with cam-actuated drive and separate guidance of **Figure 3A****.**

### Detailed Description of Example Embodiments

The present invention may be understood more readily by reference to the following detailed description of the invention taken in connection with the accompanying drawing figures, which form a part of this disclosure. It is to be understood that this invention is not limited to the specific devices, methods, conditions or parameters described and/or shown herein, and that the terminology used herein is for the purpose of describing particular embodiments by way of example only and is not intended to be limiting of the claimed invention.

Also, as used in the specification including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it will be understood that the particular value forms another embodiment.

With reference now to the drawing figures, wherein like reference numbers represent corresponding parts throughout the several views, **Figures 1A-3B** depict various embodiments of lancing devices according to example embodiments of the present invention. In general, the lancing devices herein include a lancet assembly having a housing and a needle carriage movably housed within the housing, the needle carriage adapted to support a needle thereon, the needle carriage having a first end and a second end generally opposite thereto. A needle is mounted to the needle carriage and a guide element is positioned within the housing for guiding the needle carriage for reciprocating translation. A pivotal drive cam is provided for driving the needle carriage in translation and has a cam lobe for engaging the second end of the needle carriage, wherein the needle carriage is guided by the guide element and driven in translation by the pivotal drive cam such that the guidance and drive are kept separate.

Figures 1A-3C depict a lancing device 10 with cam-actuated drive and separate guidance according to a first example embodiment of the present invention. In general, the lancing device 10 includes a housing 20 containing a cam assembly 40 and needle assembly 60. In example embodiments, the housing 20 includes a peripheral wall 24 that stretches substantially along the perimeter edge and together with a floor defines a recessed interior portion 26. The housing also includes a guide element 28 and a needle passage 23 integrally formed therein. Optionally, the wall 24 has a substantially constant thickness and the recessed interior portion preferably maintains a thickness substantially similar or greater than the wall thickness.

The guide element 28 includes a first exterior end 25 adjacent to wall 24 and a second interior end 27 located within the recessed interior portion 26. In preferred example embodiments, a needle carriage 64, having a first end 63 and a second end 65 generally opposite thereto, is movably housed within the housing 20 and is adapted to support a needle 62. As best seen in Figure 1D, the needle carriage 64 can be provided with an elongate guided surface 67 extending generally axially between the first and second ends of the carriage and the guide element 28 can include a guide surface 21 for engaging and guiding the guided surface 67 of the needle carriage 64. In one preferred embodiment, the guide element 28 within the housing 20 and the guided surface 67 of the needle carriage 64 comprise a sliding dovetail connection. Optionally the guide element 28 is a one-piece construction, but the guide element can be provided as a multi-piece construction. Also, multiple smaller guide elements can be employed, as desired.

A pivotal drive cam 42, having an outer surface or profile 42a, is provided for driving the needle carriage in translation and includes a cam lobe 44 for engaging the second end of the needle carriage. The pivotal drive cam is pivotally mounted to a pivot on a mounting pin 46 within the interior portion 26. The needle carriage 64 is guided by the guide element 28 and driven in translation by the drive cam 42 such that the guidance and drive are kept separate from one another.

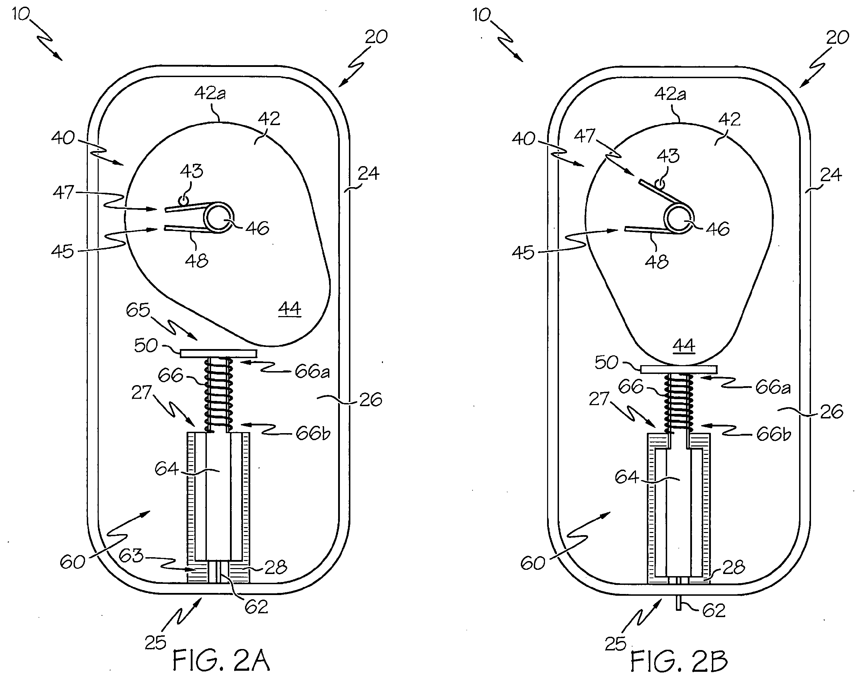

In example embodiments, the lancet device 10 can optionally include a guidance biasing spring 66 for biasing the needle carriage toward a retracted position and a separate drive spring 48 for storing potential energy for driving the pivotal drive cam 42. In preferred example embodiments, the guidance biasing spring 66, located within the guide element 28, is fixed between the first end 63 of the needle carriage 64 and guide element first end 25 (best seen in Figures 1A-C). The biasing spring is axially aligned with the needle carriage 64 and the needle 62. In other example embodiments, the biasing spring 66 is located at the second end 27 of the needle carriage 64. A proximal end 66a of spring 66, axially aligned with the needle carriage, mounts below a flat cam follower portion 50 that extends beyond the outer edge of the needle carriage and a distal end 66b of the spring is fixed at the second end 27 of guide element 28 (best seen in Figures 2A-B).

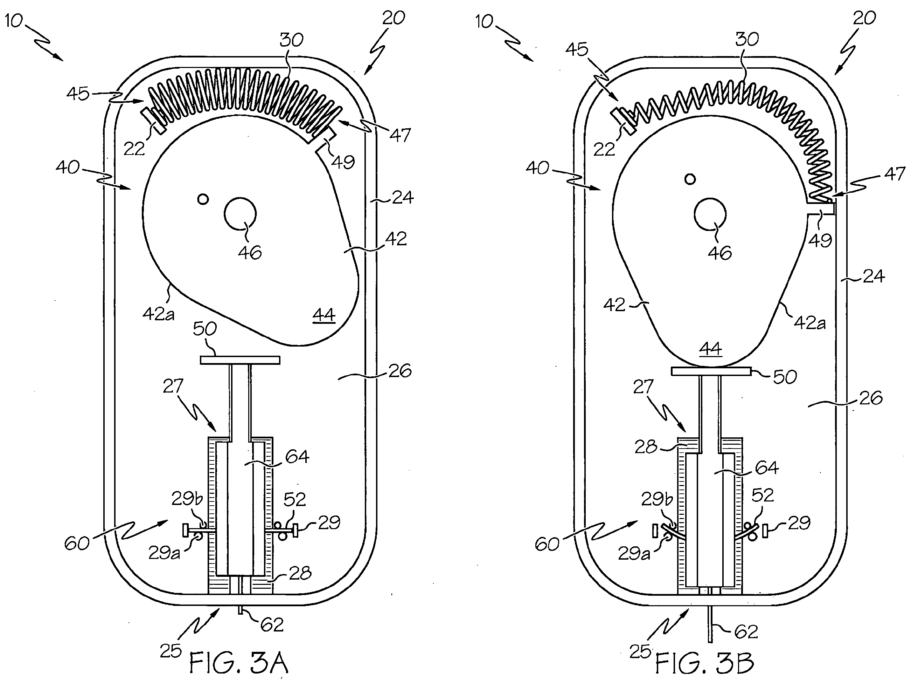

In other example embodiments, the biasing spring can comprise a leaf spring 52 fixed between the first and second ends of the guide element 28 (best seen in Figures 3A-B). The leaf spring sits perpendicular to the translational movement of the needle carriage 64 and is secured by side stops 29, front housing pins 29a, and rear housing pins 29b. Additionally, the bottom surface of the needle carriage 64 preferably includes a protrusion (not shown). The protrusion extends from the bottom surface of the needle carriage 64 towards recessed interior portion 26 and initiates contact with the biasing spring 52 when acted upon by the drive cam 22.

Referring again to Figure 1 *et seq*., the drive spring 48 has a first arm/end 45 fixed to the interior portion 26 and a second arm/end 47 fixed to the drive cam pin 43. The drive spring 48 permits potential energy to be stored for pivoting the pivotal drive cam 42 and driving the needle carriage 64 in translation. In example embodiments, the pivotal drive cam is movable between a cocked, retracted position (best seen in Figures 1A, 2A, 3A), a fired, retracted position (best seen in Figure 1 C), and an extended position between the cocked retracted position and the fired, retracted position (best seen in Figures 1B, 2B,3B). The drive spring 48 is preferably a compression spring. In additional embodiments, the pivotal drive cam 42 can be altered to receive a compression spring. Figures 3A-B depict a cam assembly 40 comprising a compression spring. The compression drive spring 30, having a first end 45 fixed to the interior spring mount 22 and a second end 47 fixed to a pivotal drive cam arm 49, additionally permits storing potential energy for pivoting the pivotal drive cam 42 and driving a needle carriage 64 in translation. Alternately, other types of springs could be used.

In example embodiments, the cam lobe 44 can be designed to achieve a variety of motions for a variety of purposes. For example, the cam lobe can be designed to have a shape that minimized the time duration of the needle exposure or that minimizes the stroke to just barely jut the needle from the end of the housing. The lobe can also be designed to control the acceleration of the needle as it enters the skin (the lobe can be designed to cause the needle to be accelerating, decelerating, or maintaining a more or less constant velocity as the needle enters the skin). For example, if the shortest skin or tissue penetration is desired, the lobe could be further pointed so that there is a very small duration of time when the lobe is engaged with the needle carriage. Alternatively, if a longer skin or tissue penetration is desired, the lobe could be further rounded and
extended so that there is a longer duration of time when the lobe is engaged with the needle carriage. By careful design of the cam lobe, the motion and acceleration of the needle into and out of the skin can be carefully controlled. Additionally, to further adjust the speed and acceleration of the needle, the mass of the cam and the carriage can be altered relative to one another to provide a desired force-to-mass ratio (and consequent acceleration as the cam lobe accelerates through its motion). Those skilled in the art will also appreciate that the drive cam spring strength also plays a role.

In operation, the cocking and releasing of the pivotal drive cam 42 can be performed in a variety of different ways. In preferred example embodiments, the cam 42 can have a recessed knob or button (not shown) that extends through the housing 20. The knob permits cocking the cam to a cocked, retracted position, and also includes a function to permit firing.

The components discussed and described herein can be formed from a variety of materials as desired by a user. In example embodiments, the lancet device and components can be formed from plastics (e.g,. polystyrene), other polymers, glass, metals, metal alloys, resins, rubbers, rubber derivatives, elastomerics (i.e. santoprene), silicones or other known materials. In preferred embodiments, the drive cam or the needle carriage may be formed from a low friction material. Materials of such low friction properties can include nylon or PTFE. Additionally, the springs can be formed from plastics, other polymers, metals, metal alloys, resins, rubbers, rubber derivatives, or other known materials.

While the invention has been described with reference to preferred and example embodiments, it will be understood by those skilled in the art that a variety of modifications, additions and deletions are within the scope of the invention, as defined by the following claims.

## Claims

1. A lancet assembly (10) comprising;
a housing (20);
a needle carriage (64) movably mounted within the housing (20), the needle carriage (64) adapted to support a needle (62) thereon, the needle carriage (64) having a first end (63) and a second end (65) generally opposite thereto, and wherein the needle carriage (64) has an elongate guided surface (67) extending generally axially between the first and second ends of the needle carriage (64);
a needle (62) mounted to the needle carriage (64);
a guidance biasing spring (66) operative for biasing the needle carriage (64) in translation towards a retracted position;
a guide element (28) positioned within the housing (20) for guiding the needle carriage (64) for reciprocating translation, the guide element (28) comprising a guide surface (21) for engaging and guiding the elongate guided surface (67) of the needle carriage (64);
a pivotal drive cam (42) for driving the needle carriage (64) in translation and having a cam lobe (44) for engaging the second end (65) of the needle carriage (64); and
a drive spring (48) for storing potential energy for pivoting the pivotal drive cam (42) and driving the needle carriage (64) in translation;
wherein the needle carriage (64) is guided by the guide element (28) and driven in translation by the pivotal drive cam (42) such that guidance of the needle carriage (64) and drive of the needle carriage (64) are kept substantially separate from one another.

2. A lancet assembly (10) as claimed in claim 1, wherein the pivotal drive cam (42) is movable between a cocked, retracted position, a fired, retracted position, and an extended position between the cocked retracted position and the fired, retracted position.

3. A lancet assembly (10) as claimed in claim 1 or 2, wherein the guidance biasing spring (66) and the drive spring (48) are separate springs from one another.

4. A lancet assembly (10) according to any preceding claim wherein the second end (65) of the needle carriage (64) comprises a cam follower portion (50) for engaging the cam lobe (44) and being driven thereby.

5. A lancet assembly as claimed in claim 1 wherein the guide element within the housing and the guided surface of the needle carriage define a sliding dovetail connection.

6. A lancet assembly (10) according to any preceding claim,
wherein the drive cam (42) and drive spring (48) form a drive mechanism for driving the needle carriage (64) in translation.

7. A lancet assembly (10) according to any preceding claim, wherein the cam lobe (44) is shaped so that, in use, a time duration of exposure of the needle (62) outside the housing (20) is minimized

8. A lancet assembly (10) according to any preceding claim, wherein the cam lobe (44) is shaped so that the acceleration of the needle (62) as it enters the skin is dependent on the shape of the lobe.

## Patentansprüche

1. Lanzettenaufbau (10), umfassend:
ein Gehäuse (20);
einen Nadelträger (64), der bewegbar im Gehäuse (20) angebracht ist, wobei der Nadelträger (64) dafür ausgelegt ist, eine Nadel (62) darauf zu lagern, wobei der Nadelträger (64) ein erstes Ende (63) und ein zweites Ende (65) hat, das im Allgemeinen dazu gegenüber ist, und wobei der Nadelträger (64) eine längliche geführte Fläche (67) hat, die sich im Allgemeinen axial zwischen dem ersten und dem zweiten Ende des Nadelträgers (64) erstreckt;
eine Nadel (62), die am Nadelträger (64) angebracht ist;
eine Spannfeder (66) zur Führung, die dafür betriebsfähig ist, den Nadelträger (64) bei der Verschiebung in eine eingezogene Position zu spannen;
ein Führungselement (28), das im Gehäuse (20) positioniert ist, um den Nadelträger (64) für eine Hin-und-her-Verschiebung zu führen, wobei das Führungselement (28) eine Führungsfläche (21) zum Ergreifen und Führen der länglichen geführten Fläche (67) des Nadelträgers (64) umfasst;
einen schwenkbaren Antriebsnocken (42) zum Antreiben des Nadelträgers (64) bei der Verschiebung und mit einer Nockenerhebung (44) zum Ergreifen des zweiten Endes (65) des Nadelträgers (64); und
eine Antriebsfeder (48) zum Speichern möglicher Energie zum Schwenken des schwenkbaren Antriebsnockens (42) und Antreiben des Nadelträgers (64) bei der Verschiebung;
wobei der Nadelträger (64) vom Führungselement (28) geführt wird und bei der Verschiebung vom schwenkbaren Antriebsnocken (42) angetrieben wird, sodass die Führung des Nadelträgers (64) und der Antrieb des Nadelträgers (64) im Wesentlichen voneinander getrennt gehalten werden.

2. Lanzettenaufbau (10) nach Anspruch 1, wobei der schwenkbare Antriebsnocken (42) zwischen einer gespannten, eingezogenen Position, einer ausgelösten, eingezogenen Position und einer ausgefahrenen Position zwischen der gespannten eingezogenen Position und der ausgelösten, eingezogenen Position bewegbar ist.

3. Lanzettenaufbau (10) nach Anspruch 1 oder 2, wobei die Spannfeder (66) zur Führung und die Antriebsfeder (48) voneinander getrennte Federn sind.

4. Lanzettenaufbau (10) nach einem der vorhergehenden Ansprüche, wobei das zweite Ende (65) des Nadelträgers (64) einen Nockenfolgerteil (50) zum Ergreifen der Nockenerhebung (44) umfasst und dadurch angetrieben wird.

5. Lanzettenaufbau nach Anspruch 1, wobei das Führungselement im Gehäuse und die geführte Fläche des Nadelträgers eine gleitende Schwalbenschwanzverbindung definieren.

6. Lanzettenaufbau (10) nach einem der vorhergehenden Ansprüche, wobei der Antriebsnocken (42) und die Antriebsfeder (48) einen Antriebsmechanismus zum Antreiben des Nadelträgers (64) bei der Verschiebung bilden.

7. Lanzettenaufbau (10) nach einem der vorhergehenden Ansprüche, wobei die Nockenerhebung (44) derart geformt ist, dass bei der Verwendung ein Zeitraum des Freiliegens der Nadel (62) außerhalb des Gehäuses (20) minimiert wird.

8. Lanzettenaufbau (10) nach einem der vorhergehenden Ansprüche, wobei die Nockenerhebung (44) derart geformt ist, dass die Beschleunigung der Nadel (62) beim Eindringen in die Haut von der Form der Erhebung abhängig ist.

## Revendications

1. Ensemble de lancette (10) comprenant :
un boîtier (20) ;
un chariot à aiguille (64) monté mobile dans le boîtier (20), le chariot à aiguille (64) étant à même de supporter une aiguille (62), le chariot à aiguille (64) ayant une première extrémité (63) et une seconde extrémité (65) qui lui est généralement opposée, et dans lequel le chariot à aiguille (64) présente une surface allongée guidée (67) s'étendant de manière générale axialement entre la première et la seconde extrémité du chariot à aiguille (64) ;
une aiguille (62) montée sur le chariot à aiguille (64) ;
un ressort de guidage à sollicitation (66) qui est à même de presser le chariot à aiguille (64) en translation vers une positon de retrait ;
un élément de guidage (28) positionné dans le boîtier (20) pour guider le chariot à aiguille (64) dans un mouvement de translation en va-et-vient, l'élément de guidage (28) comprenant une surface de guidage (21) pour s'engager sur la surface allongée guidée (67) du chariot à aiguille (64) et la guider ;
une came d'entraînement à pivotement (42) pour entraîner le chariot à aiguille (64) en translation et ayant un lobe de came (44) pour s'engager sur la seconde extrémité (65) du chariot à aiguille (64) ; et
un ressort d'entraînement (48) pour stocker de l'énergie potentielle afin de faire pivoter la came d'entraînement à pivotement (42) et entraîner le chariot à aiguille (64) en translation ;
dans lequel le chariot à aiguille (64) est guidé par l'élément de guidage (28) et entraîné en translation par la came d'entraînement à pivotement (42) de sorte que le guidage du chariot à aiguille (64) et l'entraînement du chariot à aiguille (64) soient maintenus sensiblement séparés l'un de l'autre.

2. Ensemble de lancette (10) selon la revendication 1, dans lequel la came d'entraînement à pivotement (42) est mobile entre une position rétractée armée, une position rétractée mise à feu et une position en extension entre la position rétractée armée et la position rétractée mise à feu.

3. Ensemble de lancette (10) selon la revendication 1 ou la revendication 2, dans lequel le ressort de guidage à sollicitation (66) et le ressort d'entraînement (48) sont des ressorts séparés l'un de l'autre.

4. Ensemble de lancette (10) selon l'une quelconque des revendications précédentes, dans lequel la seconde extrémité (65) du chariot à aiguille (64) comprend une partie de suiveur de came (50) pour s'engager sur le lobe de came (44) et être entraînée par celui-ci.

5. Ensemble de lancette selon la revendication 1, dans lequel l'élément de guidage à l'intérieur du boîtier et la surface guidée du chariot à aiguille définissent un raccordement en queue d'aronde coulissant.

6. Ensemble de lancette (10) selon l'une quelconque des revendications précédentes, dans lequel la came d'entraînement (42) et le ressort d'entraînement (48) forment un mécanisme d'entraînement pour entraîner le chariot à aiguille (64) en translation.

7. Ensemble de lancette (10) selon l'une quelconque des revendications précédentes, dans lequel le lobe de came (44) est conformé de sorte que, en service, la durée de l'exposition de l'aiguille (62) en dehors du boîtier (20) soit minimisée.

8. Ensemble de lancette (10) selon l'une quelconque des revendications précédentes, dans lequel le lobe de came (44) est conformé de sorte que l'accélération de l'aiguille (62) lorsqu'elle pénètre dans la peau dépende de la forme du lobe.
